# EUROPÄISCHE PATENTSCHRIFT

(11) **EP 4 138 736 B1**
(45) Veröffentlichungstag und Bekanntmachung des Hinweises auf die Patenterteilung: **12.03.2025**
(21) Anmeldenummer: 21721485.7
(22) Anmeldetag: 23.04.2021
(51) Int. Cl.: A61F 2/28, A61F 2/30, A61L 27/12, A61L 27/32, A61F 2/38

(54) **KNOCHENIMPLANTAT**
BONE IMPLANT
IMPLANT OSSEUX

(30) Priorität: 24.04.2020 DE 102020205229
(43) Veröffentlichungstag der Anmeldung: 01.03.2023
(73) Patentinhaber: Aesculap AG, 78532 Tuttlingen (DE)
(72) Erfinder: STOLINSKI, Elvira, 88605 Meßkirch (DE); BAXMANN, Marc, 31177 Harsum (DE); HOEFFGEN, Denis, 78324 Engen (DE); HETTICH, Georg, 79117 Freiburg (DE); GRUPP, Thomas, 78588 Denkingen (DE)
(74) Vertreter: Patentanwälte Ruff, Wilhelm, Beier, Dauster & Partner mbB
(86) Internationale Anmeldenummer: PCT/EP2021/060632
(87) Internationale Veröffentlichungsnummer: WO 2021/214276

(56) Entgegenhaltungen:
- EP-A1- 3 203 935
- EP-B1- 3 203 935
- US-A1- 2007 118 229
- US-A1- 2008 133 024
- US-A1- 2011 202 141
- US-A1- 2012 016 482
- US-A1- 2018 008 416
- US-A1- 2019 015 212
- US-A1- 2019 192 304

## Beschreibung

Die Erfindung betrifft ein Knochenimplantat, insbesondere zur Behandlung oder Versorgung von Knochendefekten und/oder zur Verankerung oder Fixierung eines Gelenkimplantats, vorzugsweise Kniegelenkimplantats.

Generell stellen komplexe Knochendefekte bei Revisionsoperationen an einem Gelenk eine große Herausforderung dar. Dies gilt insbesondere für die Behandlung oder Versorgung von komplexen tibialen oder femoralen Knochendefekten. Die Behandlung bzw. Versorgung solcher Knochendefekte wird zusätzlich dadurch erschwert, dass die Form und/oder Geometrie von Tibia (Schienbein) und Femur (Oberschenkelknochen) abhängig von der Größe, des Geschlechts sowie der ethnischen Herkunft eines Patienten einer natürlichen Streuung unterliegt.

Bei der Behandlung komplexer Gelenkknochendefekte werden in der Regel zwei vorrangige Ziele verfolgt, nämlich eine stabile Verankerung eines für die Behandlung bzw. Versorgung des Knochendefekts verwendeten Knochenimplantats in einem knöchernen Lager mit hoher Primärstabilität sowie eine Rekonstruktion der ursprünglichen biomechanischen Achsen.

Konventionelle Implantate haben in der Regel den Nachteil, dass sie die morphologischen Gegebenheiten, insbesondere die Form und/oder die Geometrie und/oder die Größenverhältnisse, von Knochendefekten, insbesondere tibialen oder femoralen Knochendefekten, nur eingeschränkt abbilden. Eine Folge hiervon ist, dass die Form und/oder die Geometrie und/oder die Größe des Knochendefekts an das jeweilige Implantat angepasst werden muss, um eine optimale kortikale Abstützung zu erzielen. Der hiermit einhergehende (zusätzliche) Knochenverlust erzeugt wiederum ungünstige Ausgangsbedingungen im Hinblick auf etwaige erforderlich werdende Revisionsoperationen.

In der US 2007/118229 A1 ist ein derartiges Knochenimplantat offenbart, welches einen Grundkörper in Form eines in axialer Richtung beidseitig offenen Hohlkörpers umfasst, aufweisend oder bestehend aus einem lasttragenden Material, und einen Ummantelungskörper, der den Grundkörper außenseitig wenigstens teilweise ummantelt, oder eine Vielzahl von Formkörpern, die in radialer Richtung vom Grundkörper abstehen. Der Ummantelungskörper bzw. die Formkörper weisen ein in vivo abbaubares/in vivo resorbierbares Material auf.

Aufgabe der Erfindung ist es, ein Knochenimplantat bereitzustellen, welches die eingangs im Zusammenhang von konventionellen Knochenimplantaten beschriebenen Nachteile teilweise oder vollständig vermeidet. Das Knochenimplantat soll insbesondere eine knochenschonende Behandlung oder Versorgung von Knochendefekten ermöglichen und das Risiko von Revisionsoperationen reduzieren.

Diese Aufgabe wird gelöst durch ein Knochenimplantat mit den Merkmalen gemäß unabhängigem Anspruch 1 sowie durch ein Verfahren gemäß Anspruch 14. Bevorzugte Ausgestaltungen der Erfindung sind Gegenstand der abhängigen Ansprüche 2 bis 13 sowie der vorliegenden Beschreibung.

Gemäß einem ersten Aspekt betrifft die Erfindung ein Knochenimplantat, vorzugsweise zur Behandlung oder Versorgung von Knochendefekten, insbesondere tibialen und/oder femuralen Knochendefekten, und/oder zur Fixierung oder Verankerung eine Gelenkimplantats, insbesondere Kniegelenkimplantats.

Das Knochenimplantat weist Folgendes auf:
- einen Grundkörper in Form eines in axialer Richtung beidseitig offenen Hohlkörpers, aufweisend oder bestehend aus einem lasttragenden Material,
   und
- einen Ummantelungskörper, der den Grund- oder Hohlkörper außenseitig, d.h. an einer Außenfläche des Grund- oder Hohlkörpers, wenigstens teilweise, insbesondere nur teilweise oder vollständig, ummantelt und ein in vivo abbaubares/in vivo resorbierbares Material aufweist oder aus einem in vivo abbaubaren/in vivo resorbierbaren Material besteht, oder eine Vielzahl von Formkörpern, die in radialer Richtung vom Grund- oder Hohlkörper abstehen und ein in vivo abbaubares/in vivo resorbierbares Material aufweisen oder aus einem in vivo abbaubaren/in vivo resorbierbaren Material bestehen, bestehen, wobei der Ummantelungskörper beziehungsweise die Formköper jeweils wenigstens zwei unterschiedliche in vivo abbaubare/in vivo resorbierbare Materialien aufweisen, die sich in Bezug auf ihre in vivo Abbaugeschwindigkeit/in vivo Resorptionsgeschwindigkeit voneinander unterscheiden. Dadurch ist (ebenfalls) eine zusätzliche zeitliche Steuerung der mechanischen Stabilität des Knochenimplantats sowie einer biologischen Rekonstruktion eines Knochendefektes erzielbar. Insbesondere können hierdurch die mechanische Stabilität des Knochenimplantats, die Knochenstimulierung sowie das Knochenwachstum noch besser aufeinander abgestimmt werden..

Unter dem Ausdruck "Knochenimplantat" soll im Sinne der vorliegenden Erfindung ein Implantat zur Behandlung oder Versorgung eines Knochendefektes, insbesondere eines tibialen oder femuralen Knochendefektes, und/oder zur Fixierung oder Verankerung eines Gelenkimplantats, insbesondere Kniegelenkimplantats, verstanden werden.

Unter dem Ausdruck "Formkörper" sollen im Sinne der vorliegenden Erfindung Körper oder Komponenten des Knochenimplantats verstanden werden, die dazu ausgebildet sind, eine Fixierung, Verankerung oder Abstützung des Grund- oder Hohlkörpers an/in einem Knochendefekt, insbesondere an/in einem tibialen oder femuralen Knochendefekt, zu bewirken.

Unter dem Ausdruck "Vielzahl von Formkörpern" sollen im Sinne der vorliegenden Erfindung wenigstens zwei, insbesondere mehr als zwei, Formkörper, beispielsweise zwei, drei, vier, fünf, sechs, sieben, acht, neun, zehn oder mehr Formkörper, verstanden werden.

Der Ausdruck "Knochendefekt" bedeutet im Sinne der vorliegenden Erfindung einen durch Verlust oder Beschädigung von Knochengewebe, insbesondere Gelenksknochengewebe, vorzugsweise Knie- oder Hüftgelenksknochengewebe, betroffenen Knochenbereich, insbesondere Gelenksknochenbereich, vorzugsweise Knie- oder Hüftgelenksknochenbereich. Der Knochenverlust oder die Knochenbeschädigung kann die Folge einer Knochenfraktur, eines Knochentraumas, einer Nekrose, einer Ausdünnung (beispielsweise infolge von Osteoporose), einer Knochenerkrankung wie Tumorerkrankung, einer fehlenden mechanischen Belastung (sogenanntes Stress Shielding) oder die Folge einer chirurgischen Intervention/Reintervention, insbesondere einer Revision, vorzugsweise nach einem vollständigen Knie- oder Hüftgelenkersatz, sein.

Der Ausdruck "in vivo abbaubar/in vivo resorbierbar" bedeutet im Sinne der vorliegenden Erfindung in vivo abbaubar oder in vivo resorbierbar.

Der Ausdruck "nicht in vivo abbaubar/nicht in vivo resorbierbar" bedeutet im Sinne der vorliegenden Erfindung nicht in vivo abbaubar oder nicht in vivo resorbierbar.

Der Ausdruck "vivo Abbaugeschwindigkeit/in vivo Resorptionsgeschwindigkeit" bedeutet im Sinne der vorliegenden Erfindung in vivo Abbaugeschwindigkeit oder in vivo Resorptionsgeschwindigkeit.

Unter dem Ausdruck "lasttragendes Material" soll im Sinne der vorliegenden Erfindung ein Material verstanden werden, welches in der Lage ist, in einem Knochen, wie insbesondere Schienbein und/oder Oberschenkelknochen, üblicherweise auftretenden Kräften ohne oder ohne wesentliche Deformation oder Zerstörung zu widerstehen.

Unter dem Ausdruck "in vivo abbaubares Material" soll im Sinne der vorliegenden Erfindung ein Material verstanden werden, welches in einem menschlichen oder tierischen Körper, insbesondere unter der Einwirkung von Enzymen, metabolisiert werden kann. Der Abbau des Materials kann dabei vollständig bis zur Mineralisierung, d.h. Freisetzung von chemischen Elementen und deren Einbau in anorganische Verbindungen, wie beispielsweise Kohlendioxid und/oder Sauerstoff und/oder Ammoniak, verlaufen oder auf der Stufe von abbaustabilen Zwischen- oder Transformationsprodukten stehen bleiben.

Unter dem Ausdruck "in vivo resorbierbares Material" soll im Sinne der vorliegenden Erfindung ein Material verstanden werden, welches in einem menschlichen oder tierischen Körper durch lebende Zellen oder lebendes Gewebe, wie beispielsweise Nieren, aufgenommen werden kann, ohne dass ein Abbau oder ein nennenswerter Abbau des Materials stattfindet.

Unter dem Ausdruck "tierischer Körper" soll im Sinne der vorliegenden Erfindung der Körper eines nicht humanen Säugetiers, wie beispielsweise von Pferd, Kuh, Ziege, Schaf, Schwein oder einem Nagetier, wie beispielsweise Hase, Ratte oder Maus, verstanden werden.

Der Ausdruck "in axialer Richtung" soll im Sinne der vorliegenden Erfindung entlang einer Achse, insbesondere Symmterieachse, einer Komponente oder eines Körpers, wie beispielsweise des Grund- oder Hohlkörpers und/oder des Ummantelungskörpers, des Knochenimplantats bedeuten.

Unter dem Ausdruck "Längsachse" soll im Sinne der vorliegenden Erfindung eine Achse einer Komponente oder eines Körpers, wie beispielsweise des Grund- oder Hohlkörpers und/oder des Ummantelungskörpers, des Knochenimplantats verstanden werden, welche der Richtung der größten Ausdehnung der Komponente bzw. des Körpers, wie beispielsweise des Grund- oder Hohlkörpers und/oder des Ummantelungskörpers, entspricht.

Der Ausdruck "in radialer Richtung" soll im Sinne der vorliegenden Erfindung von einem Symmetriepunkt, insbesondere Mittelpunkt, und/oder einer Symmetrieachse, insbesondere Mittelachse, einer Komponente oder eines Körpers, wie beispielsweise des Grund- oder Hohlkörpers und/oder des Ummantelungskörpers, des Knochenimplantats wegzeigend bedeuten.

Der Ausdruck "proximal" soll im Sinne der vorliegenden Erfindung zur Körpermitte zeigend oder näher zur Körpermitte gelegen bedeuten.

Der Ausdruck "distal" soll im Sinne der vorliegenden Erfindung von der Körpermitte weg zeigend oder von der Körpermitte entfernt gelegen bedeuten.

Unter dem Ausdruck "konusförmiger Hohlkörper" soll im Sinne der vorliegenden Erfindung ein Hohlkörper verstanden werden, welcher einen Mantel oder eine Mantelfläche aufweist, wobei der Mantel bzw. die Mantelfläche wenigstens abschnittsweise, insbesondere nur abschnittsweise oder vollständig, gegenüber einer Symmetrieachse, insbesondere Mittelachse, des Hohlkörpers geneigt ist.

Die Erfindung zeichnet sich insbesondere durch nachfolgende Vorteile aus:
- Durch das lasttragende Material des Grund- oder Hohlkörpers ist mit besonderem Vorteil eine unter medizinischen Gesichtspunkten ausreichende Primär- sowie Sekundärstabilität erzielbar. Unter der Primärstabilität wird allgemein die auf Reibung oder Befestigungselementen, wie beispielsweise Knochenschrauben, beruhende Fixierung eines Implantats in den ersten Wochen nach einer Operation verstanden. Unter der Sekundärstabilität wird die auf knöchernen Verwachsungen beruhende Fixierung eines Implantats verstanden. Die Sekundärstabilität setzt in der Regel etwa einen Monat nach einem operativen Eingriff ein und kann bis zu mehrere Jahre andauern.

- Ferner erlaubt das in vivo abbaubare/in vivo resorbierbare Material des Ummantelungskörpers oder der Formkörper vorteilhafterweise eine biologische Rekonstruktion eines Knochendefektes und mithin eine Verkleinerung der Knochendefektgröße. Dadurch können insbesondere verbesserte Ausgangsbedingungen für etwaige Revisionsoperationen geschaffen werden.
- Ferner ist von Vorteil, dass über die Auswahl des in vivo abbaubaren/in vivo resorbierbaren Materials des Ummantelungskörpers oder der Formkörper eine gezielte Steuerung der mechanischen Stabilität des Knochenimplantats und eine gezielte Beeinflussung der Knochenstimulierung sowie des Knochenwachstums vorgenommen werden kann. Insbesondere können hierdurch die mechanische Stabilität des Knochenimplantats, die Knochenstimulierung sowie das Knochenwachstum aufeinander abgestimmt werden.
- Das Knochenimplantat gemäß der vorliegenden Erfindung eignet sich insbesondere zur Behandlung und/oder Versorgung von tibialen und/oder femuralen Knochendekten.

In Ausgestaltung der Erfindung weist der Grund- oder Hohlkörper einen eckenlosen, insbesondere kreisförmigen, ovalen oder elliptischen, Querschnitt auf.

In weiterer Ausgestaltung der Erfindung ist der Grund- oder Hohlkörper konusförmig gestaltet. Ein konusförmig gestalteter Grund- oder Hohlkörper hat insbesondere den Vorteil, eine stabilere Verankerung eines Verlängerungsschaftes eines Gelenkimplantats zu ermöglichen. Vorzugsweise weist der Grund- oder Hohlkörper in axialer Richtung oder Längsrichtung eine sich verjüngende, insbesondere eine sich kontinuierlich oder diskontinuierlich verjüngende, Querschnittsfläche und/oder einen sich verjüngenden, insbesondere einen sich kontinuierlich oder diskontinuierlich verjüngenden, Innendurchmesser auf.

Alternativ kann der Grund- oder Hohlkörper in axialer Richtung oder Längsrichtung, insbesondere durchgehend, eine konstante Querschnittsfläche und/oder einen konstanten Innendurchmesser aufweisen.

Der Grund- oder Hohlkörper kann grundsätzlich, insbesondere in axialer Richtung oder Längsrichtung, eine konstante, d.h. gleichmäßige, oder eine variierende, d.h. ungleichmäßige, Wanddicke aufweisen. Dadurch kann mit besonderem Vorteil asymmetrisch ausgebildeten Knochendefekten entsprochen werden.

In weiterer Ausgestaltung der Erfindung weist der Grund- oder Hohlkörper eine Wanddicke von 1 mm bis 30 mm, insbesondere 5 mm bis 20 mm, vorzugsweise 8 mm bis 15 mm, auf.

Ferner kann der Grund- oder Hohlkörper, insbesondere in axialer Richtung oder Längsrichtung, eine konstante, d.h. gleichmäßige, oder eine variierende, d.h. ungleichmäßige, Form aufweisen. Dadurch kann mit besonderem Vorteil (ebenso) asymmetrisch ausgebildeten Knochendefekten entsprochen werden.

Ferner kann der Grund- oder Hohlkörper in axialer Richtung, insbesondere in Längsrichtung, eine Wandung mit einer Anzahl von Durchbrüchen oder Aussparungen, d.h. nur einem Durchbruch/eine Aussparung oder einer Vielzahl von Durchbrüchen/Aussparungen, aufweisen. Der Durchbruch/die Aussparung oder die Durchbrüche/Aussparungen kann/können insbesondere u-förmig gestaltet sein. Bevorzugt weist die Wandung des Grund- oder Hohlkörpers zwei Durchbrüche/Aussparungen, insbesondere zwei gegenüberliegend ausgebildete Durchbrüche/Aussparungen, auf. Dadurch kann mit besonderem Vorteil ein Kniegelenksimplantat in den Grund- oder Hohlkörper positioniert werden. Tibiale Komponenten von Kniegelenksimplantaten besitzen nämlich medio-lateral angebrachte "Flügel", um eine stabile Verankerung und eine Rotationsstabilität zu erhalten.

In weiterer Ausgestaltung der Erfindung handelt es sich bei dem lasttragenden Material des Grund- oder Hohlkörpers um ein in vivo nicht abbaubares/in vivo nicht resorbierbares Material. Dadurch ist mit besonderem Vorteil eine besonders effektive Primär- wie Sekundärstabilität des Knochenimplantats realisierbar.

Das in vivo nicht abbaubare/in vivo nicht resorbierbare Material ist vorzugsweise ausgewählt aus der Gruppe bestehend aus Metalle, Legierungen, Keramiken, Kunststoffe und Kombinationen, insbesondere Mischungen, davon.

Das Metall kann insbesondere ausgewählt sein aus der Gruppe bestehend aus Tantal, Titan, Eisen, Zirkonium, Niob, Gold und Platin. Besonders bevorzugt ist Titan.

Die Legierung kann insbesondere ausgewählt sein aus der Gruppe bestehend aus Magnesiumlegierung, Tantallegierung, Titanlegierung, Chrom-Cobalt-Legierung, Chrom-Cobalt-Molybdän-Legierung, Eisenlegierung, Stahl wie Edelstahl, Zirconiumlegierung, Nioblegierung, Goldlegierung, Platinlegierung und Kombinationen, insbesondere Mischungen, davon. Besonders bevorzugt ist eine Titanlegierung, insbesondere Ti-6Al-4V.

Der Kunststoff kann insbesondere ausgewählt sein aus der Gruppe bestehend Polyetherketone, Polyolefine, Polyester, Polyamide, Copolymere und Kombinationen, insbesondere Mischungen (Blends), davon.

Die Polyetherketone können insbesondere ausgewählt sein aus der Gruppe bestehend aus Polyetherketon (PEK), Polyetheretherketon (PEEK), Polyetherketonketon (PEKK), Polyetheretheretherketon (PEEEK), Polyetheretherketonketon (PEEKK), Polyetherketonetherketonketon (PEKEKK), Copolymere davon und Kombinationen, insbesondere Mischungen (Blends), davon. Besonders bevorzugt ist Polyetheretherketon (PEEK).

Die Polyolefine können insbesondere ausgewählt sein aus der Gruppe bestehend aus Polyethylen (PE), Polyethylen niedriger Dichte, Polyethylen hoher Dichte, hochmolekulares Polyethylen (HMWPE), ultrahochmolekulares Polyethylen (UHMWPE), Polypropylen (PP), Polytetrafluorethylen (PTFE), Polyvinylidenfluorid, Polyvinylidenchlorid, Polytetrafluorpropylen, Polyhexafluorpropylen, Copolymere davon und Kombinationen, insbesondere Mischungen (Blends), davon.

Die Polyester können insbesondere ausgewählt sein aus der Gruppe bestehend aus Polyethylenterephthalat, Polypropylenterephthalat, Polybutylenterephthalat, Copolymere und Kombinationen, insbesondere Mischungen (Blends), davon.

In weiterer Ausgestaltung der Erfindung ist der Ummantelungskörper als Schicht oder Beschichtung auf dem Grund- oder Hohlkörper ausgebildet.

In weiterer Ausgestaltung der Erfindung weist der Ummantelungskörper eine Dicke von 0,1 mm bis 30 mm, insbesondere 1 mm bis 10 mm, vorzugsweise 2 mm bis 5 mm, auf.

Ferner kann der Ummantelungskörper, insbesondere in axialer Richtung oder Längsrichtung, eine konstante, d.h. gleichmäßige, Dicke und/oder eine konstante, d.h. gleichmäßige, Form aufweisen.

In weiterer Ausgestaltung der Erfindung weist der Ummantelungskörper, insbesondere in axialer Richtung oder Längsrichtung, eine variierende, d.h. ungleichmäßige, Form und/oder Dicke auf. Dadurch kann mit besonderem Vorteil (ebenfalls) asymmetrisch ausgebildeten Knochendefekten entsprochen werden.

In weiterer Ausgestaltung der Erfindung ist die Dicke des Ummantelungskörpers, insbesondere in axialer Richtung oder Längsrichtung, an einem Ende des Ummantelungskörpers größer als einem anderen Ende des Ummantelungskörpers und/oder nimmt die Dicke des Ummantelungskörpers, insbesondere in axialer Richtung oder Längsrichtung, in Richtung eines Endes des Ummantelungskörpers, insbesondere kontinuierlich oder diskontinuierlich, zu und/oder nimmt die Dicke des Ummantelungskörpers, insbesondere in axialer Richtung oder Längsrichtung, von einem Ende des Ummantelungskörpers zu einem anderen Ende des Ummantelungskörpers, insbesondere kontinuierlich oder diskontinuierlich, zu.

Handelt es ich bei dem Knochenimplantat um ein tibiales Implantat, d.h. um ein Implantat zur Behandlung oder Versorgung eines tibialen Knochendefektes, ist die Dicke des Ummantelungskörpers, insbesondere in axialer Richtung oder Längsrichtung, vorzugsweise an einem distalen Ende des Ummantelungskörpers größer als an einem proximalen Ende des Ummantelungskörpers und/oder nimmt die Dicke des Ummantelungskörpers, insbesondere in axialer Richtung oder Längsrichtung, vorzugsweise in Richtung eines distalen Endes des Ummantelungskörpers, insbesondere kontinuierlich oder diskontinuierlich, zu und/oder nimmt die Dicke des Ummantelungskörpers, insbesondere in axialer Richtung oder Längsrichtung, vorzugsweise von einem proximalen Ende des Ummantelungskörpers zu einem distalen Ende des Ummantelungskörpers, insbesondere kontinuierlich oder diskontinuierlich, zu.

Handelt es ich bei dem Knochenimplantat um ein femurales Implantat, d.h. um ein Implantat zur Behandlung oder Versorgung eines femuralen Knochendefektes, ist die Dicke des Ummantelungskörpers, insbesondere in axialer Richtung oder Längsrichtung, vorzugsweise an einem proximalen Ende des Ummantelungskörpers größer als an einem distalen Ende des Ummantelungskörpers und/oder nimmt die Dicke des Ummantelungskörpers, insbesondere in axialer Richtung oder Längsrichtung, vorzugsweise in Richtung eines proximalen Endes des Ummantelungskörpers, insbesondere kontinuierlich oder diskontinuierlich, zu und/oder nimmt die Dicke des Ummantelungskörpers, insbesondere in axialer Richtung oder Längsrichtung, vorzugsweise von einem distalen Ende des Ummantelungskörpers zu einem proximalen Ende des Ummantelungskörpers, insbesondere kontinuierlich oder diskontinuierlich, zu.

In weiterer Ausgestaltung der Erfindung ist der Grund- oder Hohlkörper außenseitig teilweise nicht von dem Ummantelungskörper umgeben.

In weiterer Ausgestaltung der Erfindung weist der Grund- oder Hohlkörper eine poröse, insbesondere offenporige, und/oder mikrostrukturierte Oberfläche auf. Hierdurch kann eine Osteointegration, d.h. ein An- oder Einwachsen von natürlichem Knochengewebe, induziert werden. Dies wiederum begünstigt mit besonderem Vorteil eine stabile Fixierung oder Verankerung des Grundkörpers und mithin des Knochenimplantats an/in einem Knochendefekt. Dadurch sind insbesondere verbesserte Sekundärstabilitäten erreichbar. Ferner bewirkt das An- oder Einwachsen von Knochengewebe in vorteilhafter Weise eine Knochendefektverkleinerung.

Bevorzugt weist die poröse, insbesondere offenporige, Oberfläche des Grund- oder Hohlkörpers Poren mit einem Durchmesser, insbesondere mittleren Durchmesser, bestimmt mittels Rasterelektronenmikroskopie und/oder Quecksilber-Porosimetrie, von 10 µm bis 400 µm, insbesondere 50 µm bis 200 µm, bevorzugt 100 µm bis 135 µm, auf. Die in diesem Absatz offenbarten Porendurchmesser sind im Hinblick auf eine Osteointegration des Grund- oder Hohlkörpers besonders vorteilhaft.

Ferner können Poren, insbesondere der Oberfläche, des Grund- oder Hohlkörpers mit einem in vivo abbaubaren/in vivo resorbierbaren Material befüllt sein. Die Poren können insbesondere nur teilweise oder vollständig mit dem in vivo abbaubaren/in vivo resorbierbaren Material befüllt sein. Vorzugsweise sind das in vivo abbaubare/in vivo resorbierbare Material, das die Poren, insbesondere der Oberfläche, des Grund- oder Hohlkörpers befüllt, und das in vivo abbaubare/in vivo resorbierbare Material des Ummantelungskörpers oder der Vielzahl von Formkörpern voneinander verschieden. Dadurch kann eine mehrstufige, beispielsweise zweistufige, Abbau- oder Resorptionskinetik des Knochenimplantats erzeugt werden. Dies stellt vorteilhafterweise eine weitere Möglichkeit dar, die mechanische Stabilität des Knochenimplantats, die Knochenstimulierung sowie das Knochenwachstum aufeinander abzustimmen. Bezüglich geeigneter in vivo abbaubarer/in vivo resorbierbarer Materialien sei auf die in der nachfolgenden Beschreibung im Zusammenhang des Ummantelungskörpers sowie der Vielzahl von Formkörpern offenbarten in vivo abbaubaren/in vivo resorbierbaren Materialien Bezug genommen.

Die mikrostrukturierte Oberfläche des Grund- oder Hohlkörpers kann beispielsweise von einer Metallstruktur, insbesondere Metallgitterstruktur, gebildet sein. Vorzugsweise handelt es sich bei der Metallstruktur, insbesondere Metallgitterstruktur, um eine Titanstruktur, insbesondere Titangitterstruktur. Eine entsprechende Titanstruktur ist beispielsweise unter der Bezeichnung Structan^{®} kommerziell erhältlich. Bezüglich weiterer geeigneter Materialien für den Grund- oder Hohlkörper wird auf die bisherigen im Zusammenhang des Grund- oder Hohlkörpers beschriebenen Materialien Bezug genommen.

Unter dem Ausdruck "mikrostrukturierte Oberfläche" soll im Sinne der vorliegenden Erfindung eine Oberfläche mit Strukturelementen, insbesondere in Form von Vertiefungen und/oder Erhebungen, verstanden werden, wobei die Strukturelemente Abmessungen, beispielsweise Tiefen und/oder Höhen, im Mikrometermaßstab besitzen.

Beispielsweise kann die mikrostrukturierte Oberfläche des Grund- oder Hohlkörpers Vertiefungen mit einer Tiefe von 20 µm bis 1000 µm, insbesondere 100 µm bis 600 µm, bevorzugt 300 µm bis 400 µm, und/oder Erhebungen mit einer Höhe von 20 µm bis 1000 µm, insbesondere 100 µm bis 600 µm, bevorzugt 300 µm bis 400 µm, aufweisen.

Ferner kann die Oberfläche des Grund- oder Hohlkörpers von einer Beschichtung des Grund- oder Hohlkörpers gebildet sein. Mit anderen Worten kann es sich bei der Oberfläche des Grund- oder Hohlkörpers um eine Oberfläche einer Beschichtung, welche den Grund- oder Hohlkörper beschichtet oder bedeckt, handeln. Die Beschichtung kann den Grund- oder Hohlkörper wenigstens teilweise, insbesondere nur teilweise oder vollständig, beschichten oder bedecken. Ferner kann die Beschichtung eine Dicke von 0,01 µm bis 1000 µm, insbesondere 1 µm bis 100 µm, vorzugsweise 10 µm bis 50 µm, aufweisen. Ferner kann die Beschichtung eine Porosität von 20 % bis 90 %, insbesondere 50 % bis 85 %, bevorzugt 60 % bis 80 %, aufweisen. Bevorzugt weist die Beschichtung Titan, d.h. elementares oder metallisches Titan, oder ein Calciumphosphat, vorzugsweise Hydroxylapatit, auf. Beispielsweise kann es sich bei der Beschichtung um eine reine Titanbeschichtung, d.h. um eine aus Titan bestehende Beschichtung, handeln. Eine entsprechende Beschichtung kann beispielsweise mittels eines von der Anmelderin unter der Bezeichnung Plasmapore^{®} durchgeführten Verfahrens hergestellt werden. Hierbei wird reines Titanpulver mittels eines Plasmabeschichtungsprozesses unter Vakuum oder Unterdruck mit einer Dicke von 150 µm und einer Porosität von 35 % bis 60 % auf ein zu beschichtendes Substrat aufgebracht. Bezüglich weiterer geeigneter Materialien für die Beschichtung wird auf die bisherigen im Zusammenhang des Grund- oder Hohlkörpers beschriebenen Materialien Bezug genommen.

In weiterer Ausgestaltung der Erfindung weist der Ummantelungskörper eine poröse, insbesondere offenporige, und/oder mikrostrukturierte Oberfläche auf. Hierdurch kann eine Osteointegration (ebenfalls) induziert und die Sekundärstabilität des Knochenimplantats verbessert sowie abhängig vom Material des Ummantelungskörpers eine Reduzierung einer Knochendefektgröße erzielt werden.

Bevorzugt weist die poröse, insbesondere offenporige, Oberfläche des Ummantelungskörpers Poren mit einem Durchmesser, insbesondere mittleren Durchmesser, bestimmt mittels Rasterelektronenmikroskopie und/oder Quecksilber-Porosimetrie, von 20 µm bis 1000 µm, insbesondere 50 µm bis 500 µm, bevorzugt 100 µm bis 300 µm, auf. Die in diesem Absatz offenbarten Porendurchmesser sind im Hinblick auf eine Osteointegration des Ummantelungskörpers besonders vorteilhaft.

Ferner können Poren, insbesondere der Oberfläche, des Ummantelungskörpers mit einem in vivo abbaubaren/in vivo resorbierbaren Material befüllt sein. Die Poren können insbesondere nur teilweise oder vollständig mit dem in vivo abbaubaren/in vivo resorbierbaren Material befüllt sein. Vorzugsweise sind das in vivo abbaubare/in vivo resorbierbare Material, das die Poren, insbesondere der Oberfläche, des Ummantelungskörpers befüllt, und das in vivo abbaubare/in vivo resorbierbare Material des Ummantelungskörpers voneinander verschieden. Dadurch lässt sich eine mehrstufige, beispielsweise zweistufige, Abbau- oder Resorptionskinetik des Knochenimplantats ausbilden. Dies stellt vorteilhafterweise eine weitere Möglichkeit dar, die mechanische Stabilität des Knochenimplantats, die Knochenstimulierung sowie das Knochenwachstum aufeinander abzustimmen. Bezüglich geeigneter in vivo abbaubarer/in vivo resorbierbarer Materialien sei auf die in der nachfolgenden Beschreibung im Zusammenhang des Ummantelungskörpers offenbarten in vivo abbaubaren/in vivo resorbierbaren Materialien Bezug genommen.

Ferner kann die mikrostrukturierte Oberfläche des Ummantelungskörpers Vertiefungen mit einer Tiefe von 20 µm bis 1000 µm, insbesondere 100 µm bis 600 µm, bevorzugt 300 µm bis 400 µm, und/oder Erhebungen mit einer Höhe von 20 µm bis 1000 µm, insbesondere 100 µm bis 600 µm, bevorzugt 300 µm bis 400 µm, aufweisen.

Ferner kann die Oberfläche des Ummantelungskörpers von einer Beschichtung des Ummantelungskörpers gebildet sein. Mit anderen Worten kann es sich bei der Oberfläche des Ummantelungskörpers um eine Oberfläche einer Beschichtung, welche den Ummantelungskörper beschichtet oder bedeckt, handeln. Die Beschichtung kann den Ummantelungskörper wenigstens teilweise, insbesondere nur teilweise oder vollständig, beschichten oder bedecken. Ferner kann die Beschichtung eine Dicke von 0,01 µm bis 1000 µm, insbesondere 1 µm bis 100 µm, vorzugsweise 10 µm bis 50 µm, aufweisen. Ferner kann die Beschichtung eine Porosität von 20 % bis 90 %, insbesondere 50 % bis 85 %, bevorzugt 60 % bis 80 %, aufweisen. Bezüglich geeigneter Materialien für die Beschichtung wird auf die im Folgenden noch im Zusammenhang des Ummantelungskörpers beschriebenen Materialien Bezug genommen.

In weiterer Ausgestaltung der Erfindung weisen die Formkörper oder nur ein Teil der Formkörper eine poröse, insbesondere offenporige, und/oder mikrostrukturierte Oberfläche auf.

Insbesondere kann die poröse, insbesondere offenporige, Oberfläche der Formkörper Poren mit einem Durchmesser, insbesondere mittleren Durchmesser, bestimmt mittels Rasterelektronenmikroskopie und/oder Quecksilber-Porosimetrie, von 50 µm bis 900 µm, insbesondere 150 µm bis 700 µm, besonders bevorzugt 300 µm bis 400 µm, aufweisen. Die mikrostrukturierte Oberfläche der Formkörper kann Vertiefungen mit einer Tiefe von 50 µm bis 900 µm, insbesondere 150 µm bis 700 µm, bevorzugt 300 µm bis 400 µm, und/oder Erhebungen mit einer Höhe von 50 µm bis 900 µm, insbesondere 150 µm bis 700 µm, bevorzugt 300 µm bis 400 µm, aufweisen. Dadurch ist mit besonderem Vorteil (ebenso) eine Osteointegration und abhängig vom Material der Formkörper auch eine biologische Rekonstruktion und mithin Verkleinerung eines zu behandelnden oder zu versorgenden Knochendefekts erzielbar.

Ferner können Poren, insbesondere der Oberfläche, der Formköper mit einem in vivo abbaubaren/in vivo resorbierbaren Material befüllt sein. Die Poren können insbesondere nur teilweise oder vollständig mit dem in vivo abbaubaren/in vivo resorbierbaren Material befüllt sein. Vorzugsweise sind das in vivo abbaubare/in vivo resorbierbare Material, das die Poren, insbesondere der Oberfläche, der Formkörper befüllt, und das in vivo abbaubare/in vivo resorbierbare Material der Formkörper voneinander verschieden. Dadurch kann eine mehrstufige, beispielsweise zweistufige, Abbau- oder Resorptionskinetik des Knochenimplantats erzeugt werden. Dies stellt vorteilhafterweise eine weitere Möglichkeit dar, die mechanische Stabilität des Knochenimplantats, die Knochenstimulierung sowie das Knochenwachstum aufeinander abzustimmen. Bezüglich geeigneter in vivo abbaubarer/in vivo resorbierbarer Materialien sei auf die in der nachfolgenden Beschreibung im Zusammenhang der Formkörper offenbarten in vivo abbaubaren/in vivo resorbierbaren Materialien Bezug genommen.

Ferner kann die poröse, insbesondere offenporige, Oberfläche der Formkörper oder eines Teils der Formkörper von einer Beschichtung der Formkörper gebildet sein. Die Beschichtung kann die Formkörper wenigstens teilweise, insbesondere nur teilweise oder vollständig, beschichten oder bedecken. Ferner kann die Beschichtung eine Dicke von 0,01 µm bis 1000 µm, insbesondere 1 µm bis 100 µm, vorzugsweise 10 µm bis 50 µm, aufweisen. Ferner kann die Beschichtung eine Porosität von 20 % bis 90 %, insbesondere 50 % bis 85 %, bevorzugt 50 % bis 70 %, aufweisen. Bezüglich weiterer geeigneter Materialien für die Beschichtung wird auf die im Folgenden noch im Zusammenhang der Formkörper beschriebenen Materialien Bezug genommen.

Ferner kann die mikrostrukturierte Oberfläche der Formkörper oder eines Teils der Formkörper Vertiefungen mit einer Tiefe von 50 µm bis 900 µm, insbesondere 150 µm bis 700 µm, bevorzugt 300 µm bis 400 µm, und/oder Erhebungen mit einer Höhe von 50 µm bis 900 µm, insbesondere 150 µm bis 700 µm, bevorzugt 300 µm bis 400 µm, aufweisen.

Ferner können die Formkörper eine Dicke, d.h. Wanddicke oder Höhe, insbesondere eine konstante, d.h. gleichmäßige, oder variierende, d.h. ungleichmäßige, Dicke, d.h. Wanddicke oder Höhe, von 1 mm bis 30 mm, insbesondere 5 mm bis 20 mm, vorzugsweise 8 mm bis 15 mm, aufweisen.

Vorteilhafterweise kann sowohl über die Dicke der Formkörper oder die Dicke des Ummantelungskörpers als auch über die Wanddicke des Grund- oder Hohlkörpers die Gesamtwanddicke des Knochenimplantats gezielt und mithin anwendungsorientiert gesteuert werden.

Weiter sind die Formkörper vorzugsweise als Flächengebilde gestaltet, d.h. die Formkörper weisen vorzugsweise eine Länge und eine Breite auf, welche größer sind als eine Dicke (Höhe) der Formkörper. Bevorzugt sind die Formkörper platten- und/oder scheibenförmig gestaltet. Insbesondere können die Formkörper jeweils zwei gegenüberliegend angeordnete Hauptflächen aufweisen, welche jeweils von zwei gegenüberliegend angeordneten Querseitenflächen sowie von zwei gegenüberliegend angeordneten Längsseitenflächen begrenzt sind. Grundsätzlich können die Querseitenflächen plan, d.h. eben oder nicht gewölbt, und/oder gewölbt, insbesondere nach außen gewölbt, sein. Weiter können die Längsseitenflächen grundsätzlich plan, d.h. eben oder nicht gewölbt, und/oder gewölbt, insbesondere nach außen gewölbt, sein. Zum Beispiel können die Formkörper jeweils eine gewölbte, insbesondere nach außen gewölbte, Längsseitenfläche und im Übrigen plane Seitenflächen, d.h. eine plane Längsseitenfläche und zwei plane Querseitenflächen, aufweisen.

Ferner können die Formkörper oder kann ein Teil der Formkörper jeweils wenigstens eine Sollbruchlinie, insbesondere nur eine Sollbruchlinie oder eine Vielzahl von Sollbruchlinien, aufweisen. Die Sollbruchlinie/Sollbruchlinien erstreckt/erstrecken sich vorzugsweise in Längsrichtung der Formkörper. Die Sollbruchlinie/Sollbruchlinien können beispielsweise als Perforationen, Kerben oder Ritzspuren gestaltet sein. Dadurch ist eine zusätzliche Anpassung der Formkörper und mithin des Knochenimplantats an patientenindividuelle Knochendefekte erzielbar. Insbesondere kann hierdurch (zusätzlich) ein Knochenverlust durch Anpassung eines Knochendefekts an das Knochenimplantat reduziert oder vollständig vermieden werden.

In weiterer Ausgestaltung der Erfindung ist das in vivo abbaubare/in vivo resorbierbare Material, insbesondere sind die wenigstens zwei unterschiedlichen in vivo abbaubaren/in vivo resorbierbaren Materialien, ausgewählt aus der Gruppe bestehend aus Metalle, Polyhydroxyalkanoate, Polycaprolacton, Poly-p-dioxanon (Poly-1,4-dioxan-2-on), Polymethylenterephthalat, Calciumphosphate, Calciumsulfate, Calciumoxid (CaO), Calciumhydroxid (Ca(OH)₂), Calciumcarbonat (CaCO₃), Calciumcitrat, Calciumlactat, Calciumacetat, Calciumtartrat, Calciumchlorid (CaCl₂), Magnesiumphosphate, Magnesiumsulfate, Magnesiumhydroxid (Mg(OH)₂), Magnesiumhydroxidcarbonat (beispielsweise als 4 MgCO₃ x Mg(OH)₂ x 5 H₂O), Magnesiumoxid (MgO), Magnesiumcitrate (Mg₃(C₆H₅O₇)₂) oder Mg(C₆H₆O₇)), Calcium-Magnesiumcarbonat (CaMg(CO₃)₂ und Kombinationen, insbesondere Mischungen, davon.

Bei den Metallen handelt es sich vorzugsweise um Magnesium.

Die Polyhydroxyalkanoate können insbesondere ausgewählt sein aus der Gruppe bestehend aus Polylactid, Polyglycolid, Poly-3-hydroxybutyrat, Poly-4-hydroxybutyrat, Copolymere davon und Kombinationen, insbesondere Mischungen (Blends), davon.

Die Calciumphosphate können insbesondere ausgewählt sein aus der Gruppe bestehend aus Tricalciumphosphat (TCP) wie α-Tricalciumphosphat (α-TCP) und/oder β-Tricalciumphosphat (β-TCP), Apatite wie Hydroxylapatit (HA), Calcium-defizientes Hydroxylapatit (CdHA), substituiertes Hydroxylapatit, nicht stöchiometrisches Hydroxylapatit, nanoskaliges Hydroxylapatit, Tetracalciumphosphat (TTCP), Monocalciumphosphat-Monohydrat (MCPM), Monocalciumphosphat-Anhydrid (MCPA), Dicalciumphosphat-Anhydrid (DCPA), Dicalciumphosphat-Dihydrat (DCPD), Octacalciumphosphat (OCP), amorphes Calciumphosphat (ACP), Calciumglycerophosphat und Kombinationen, insbesondere Mischungen, davon.

Die Calciumsulfate können insbesondere ausgewählt sein aus der Gruppe bestehend aus Calciumsulfat (CaSO₄), Calciumsulfat-Hemihydrat (CaSO₄ x 0.5 H₂O), Calciumsulfat-Dihydrat (CaSO₄ x 2 H₂O) und Kombinationen, insbesondere Mischungen, davon.

Die Magnesiumphosphate können insbesondere ausgewählt sein aus der Gruppe bestehend aus Magnesiumhydrogenphosphat (MgHPO₄) in Form der Hydrate oder als wasserfreie Substanz, Trimagnesiumphosphat (Mg₃(PO₄)₂), Magnesiumdihydrogenphosphat (Mg(H₂PO₄)₂) in Form der Hydrate oder als wasserfreie Substanz, Magnesiumglycerophosphat und Kombinationen, insbesondere Mischungen, davon.

Besonders bevorzugt handelt es sich bei dem in vivo abbaubaren/in vivo resorbierbaren Material um Tricalciumphosphat. Das Tricalciumphosphat kann in kristalliner Form vorliegen. Insbesondere kann das Tricalciumphosphat eine Kristallinität von 50 % bis 99 %, insbesondere 75 % bis 95 %, aufweisen. Ferner kann es sich bei dem Tricalciumphosphat um ein mikrokristallines Tricalciumphosphat, d.h. um Tricalciumphosphat mit Kristalliten, welche wenigstens eine Abmessung oder Dimension im Mikrometerbereich, insbesondere in einem Bereich von > 0,5 µm, insbesondere 0,6 µm bis 500 µm, bevorzugt 0,6 µm bis 100 µm, aufweisen, handeln. Bei der wenigstens einen Abmessung bzw. Dimension kann es sich um die Länge und/oder die Breite und/oder die Dicke bzw. Höhe und/oder den Durchmesser, insbesondere mittleren Durchmesser, der Kristallite handeln. Alternativ kann es sich bei dem Tricalciumphosphat um ein nanokristallines Tricalciumphosphat, d.h. um Tricalciumphosphat mit Kristalliten, welche wenigstens eine Abmessung oder Dimension im Nanometerbereich, insbesondere in einem Bereich von 0,1 nm bis 500 nm, bevorzugt 0,1 nm bis 100 nm, aufweisen, handeln. Bei der wenigstens einen Abmessung bzw. Dimension kann es sich um die Länge und/oder die Breite und/oder die Dicke bzw. Höhe und/oder den Durchmesser, insbesondere mittleren Durchmesser, der Kristallite handeln. Alternativ kann es sich bei dem Tricalciumphosphat um ein makrokristallines Tricalciumphosphat handeln. Alternativ kann das Tricalciumphosphat in amorpher Form vorliegen. Weiter kann es sich bei dem Tricalciumphosphat um ein phasenreines Tricalciumphosphat handeln. Unter dem Ausdruck "phasenrein" soll an dieser Stelle insbesondere phasenrein im Sinne einer einschlägigen Vorschrift, vorzugsweise nach ASTM F1088, verstanden werden. Ferner kann das Tricalciumphosphat eine Porosität < 50 %, insbesondere < 20 %, vorzugsweise < 15 %, aufweisen. Alternativ kann das Tricalciumphosphat nicht porös ausgebildet sein. Vorzugsweise ist das Tricalciumphosphat ausgewählt aus der Gruppe bestehend aus α-Tricalciumphosphat (α-TCP), β-Tricalciumphosphat (β-TCP) und eine Mischung davon. Insbesondere bevorzugt handelt es sich bei dem Tricalciumphosphat um ein gesintertes Tricalciumphosphat, vorzugsweise ausgewählt aus der Gruppe bestehend aus gesintertes α-Tricalciumphosphat (gesintertes α-TCP), gesintertes β-Tricalciumphosphat (gesintertes β-TCP) und eine Mischung davon.

Alternativ kann es sich bei dem in vivo abbaubaren/in vivo resorbierbaren Material insbesondere um Hydroxylapatit handeln. Der Hydroxylapatit kann in kristalliner Form vorliegen. Insbesondere kann der Hydroxylapatit eine Kristallinität von 50 % bis 99 %, insbesondere 75 % bis 95 %, aufweisen. Ferner kann es sich bei dem Hydroxylapatit um einen mikrokristallinen Hydroxylapatit, d.h. um einen Hydroxylapatit mit Kristalliten, welche wenigstens eine Abmessung oder Dimension im Mikrometerbereich, insbesondere in einem Bereich von > 0,5 µm, insbesondere 0,6 µm bis 500 µm, bevorzugt 0,6 µm bis 100 µm, aufweisen, handeln. Bei der wenigstens einen Abmessung bzw. Dimension kann es sich um die Länge und/oder die Breite und/oder die Dicke bzw. Höhe und/oder den Durchmesser, insbesondere mittleren Durchmesser, der Kristallite handeln. Alternativ kann es sich bei dem Hydroxylapatit um einen nanokristallinen Hydroxylapatit, d.h. um einen Hydroxylapatit mit Kristalliten, welche wenigstens eine Abmessung oder Dimension im Nanometerbereich, insbesondere in einem Bereich von 0,1 nm bis 500 nm, bevorzugt 0,1 nm bis 100 nm, aufweisen, handeln. Bei der wenigstens einen Abmessung bzw. Dimension kann es sich um die Länge und/oder die Breite und/oder die Dicke bzw. Höhe und/oder den Durchmesser, insbesondere mittleren Durchmesser, der Kristallite handeln. Alternativ kann es sich bei dem Hydroxylapatit um ein makrokristallines Hydroxylapatit handeln. Weiter kann es sich bei dem Hydroxylapatit um einen phasenreinen Hydroxylapatit handeln. Unter dem Ausdruck "phasenrein" soll in diesem Zusammenhang insbesondere phasenrein im Sinne einer einschlägigen Vorschrift, vorzugsweise nach ASTM F1185, verstanden werden. Ferner kann der Hydroxylapatit eine Porosität < 50 %, insbesondere < 20 %, vorzugsweise < 15 %, aufweisen. Durch eine geringe Porosität kann eine hohe mechanische Stabilität erreicht werden. Alternativ kann der Hydroxylapatit nicht porös ausgebildet sein. Bei dem Hydroxylapatit kann es sich ferner insbesondere um einen gesinterten Hydroxylapatit handeln.

Ferner können/kann der Ummantelungskörper und/oder die Formkörper oder ein Teil der Formkörper ein in vivo nicht abbaubares/in vivo nicht resorbierbares Material aufweisen oder aus einem solchen Material bestehen. Bezüglich geeigneter in vivo nicht abbaubarer/in vivo nicht resorbierbarer Materialien wird auf die vorangegangenen im Zusammenhang des Grund- oder Hohlkörpers erwähnten Materialien Bezug genommen.

Ferner können/kann der Ummantelungskörper und/oder die Formkörper oder ein Teil der Formkörper sowohl ein in vivo abbaubares/in vivo resorbierbares Material als auch ein in vivo nicht abbaubares/in vivo nicht resorbierbares Material aufweisen oder sowohl aus einem in vivo abbaubaren/in vivo resorbierbaren Material als auch aus einem in vivo nicht abbaubaren/in vivo nicht resorbierbaren Material bestehen. Bezüglich geeigneter in vivo abbaubarer/in vivo resorbierbarer Materialien sowie vivo nicht abbaubarer/in vivo nicht resorbierbarer Materialien wird vollständig auf die in der bisherigen Beschreibung offenbarten Materialien Bezug genommen.

Ferner kann das Knochenimplantat mittels eines additiven oder generativen Fertigungsverfahrens hergestellt sein. Insbesondere können/kann der Ummantelungskörper und/oder die Formkörper mittels eines additiven oder generativen Fertigungsverfahrens außenseitig, d.h. an einer Außenfläche des Grund- oder Hohlkörpers, an dem Grund- oder Hohlkörper angeformt oder angefügt sein. Bezüglich weiterer Merkmale und Vorteile eines solchen Verfahrens wird vollumfänglich auf die nachfolgenden im Rahmen des zweiten Erfindungsaspekts gemachten Ausführungen Bezug genommen.

Gemäß einem zweiten Aspekt betrifft die Erfindung ein Verfahren zum Herstellen eines Knochenimplantats, insbesondere eines Knochenimplantats gemäß erstem Erfindungsaspekt.

Bei dem Verfahren wird das Knochenimplantat mittels eines additiven oder generativen Fertigungsverfahrens, d.h. mittels 3D-Druck, hergestellt.

Alternativ oder in Kombination wird ein Grundkörper in Form eines in axialer Richtung beidseitig offenen oder geöffneten Hohlkörpers, der ein lasttragendes Material aufweist oder aus einem lasttragenden Material besteht, mittels des/eines additiven oder generativen Fertigungsverfahrens, d.h. mittels 3D-Druck, wenigstens teilweise, insbesondere nur teilweise oder vollständig, mit einem Ummantelungskörper, aufweisend oder bestehend aus einem in vivo abbaubaren/in vivo resorbierbaren Material, ummantelt, insbesondere stoffschlüssig ummantelt, oder mit einer Vielzahl von Formkörpern, die ein in vivo abbaubares/in vivo resorbierbares Material aufweisen oder aus einem in vivo abbaubaren/in vivo resorbierbaren Material bestehen, derart verbunden, insbesondere derart stoffschlüssig verbunden, werden, dass die Formkörper in radialer Richtung von dem Grundkörper abstehen oder abragen. Der Grund- oder Hohlkörper ist vorzugsweise konusförmig gestaltet.

Unter dem Ausdruck "additives Fertigungsverfahren" bzw. "generatives Fertigungsverfahren" bzw. "3D-Druck" sollen im Sinne der vorliegenden Erfindung Verfahren zur schnellen und kostengünstigen Fertigung von Modellen, Mustern, Prototypen, Werkzeugen, Halbfabrikaten und Endprodukten wie insbesondere Knochenimplantaten verstanden werden. Diese Verfahren werden häufig auch als "Rapid Prototyping" bezeichnet. Die Fertigung erfolgt direkt auf der Basis von rechnerinternen Datenmodellen aus insbesondere formlosem Material, beispielweise aus Flüssigkeiten, Schmelzen, Pulvern oder Ähnlichem, oder formneutralem Material, beispielsweise aus band- oder drahtförmigem Material, mittels chemischer und/oder physikalischer Prozesse.

Ein generatives bzw. additives Fertigungsverfahren bietet insbesondere den Vorteil, das Knochenimplantat in Bezug auf Materialien und/oder Form und/oder Geometrie und/oder Oberflächenstruktur gezielt zu beeinflussen.

Das generative Fertigungsverfahren kann insbesondere ausgewählt sein aus der Gruppe bestehend aus Pulverbettverfahren, Freiraumverfahren und Flüssigmaterialverfahren.

Das Pulverbettverfahren kann ausgewählt sein aus der Gruppe bestehend aus selektives Laserschmelzen, selektives Lasersintern, selektives Hitzesintern (Selective Heat Sintering), Verfestigen von Pulvermaterial mittels Binder (Binder Jetting) und Elektronenstrahlschmelzen.

Das Freiraumverfahren kann ausgewählt sein aus der Gruppe bestehend aus Schmelzschichtung (Fused Deposition Modeling), LOM-Verfahren (Laminated-Object-Modeling-Verfahren), Auftragsschweißen (Cladding), Wachs-Depositions-Modellierung (Wax Deposition Modeling), Contour Grafting, Kaltgasspritzen und Elektronenstrahlschmelzen.

Das Flüssigmaterialverfahren kann ausgewählt sein aus der Gruppe bestehend aus Stereolithografie, DLP-Verfahren (Digital-Light-Processing-Verfahren) und LCM-Verfahren. Das LCM-Verfahren kann ein Liquid-Composite-Molding-Verfahren oder ein Lithography-based-Ceramic-Manufacturing-Verfahren sein.

Bezüglich weiterer Merkmale und Vorteile des Verfahrens, insbesondere des Knochenimplantats, des Grundkörpers, des Ummantelungskörpers sowie der Formkörper, wird vollständig auf die im Rahmen des ersten Erfindungsaspekts gemachten Ausführungen Bezug genommen. Die dort insoweit gemachten Ausführungen gelten sinngemäß auch für das Verfahren gemäß zweitem Erfindungsaspekt.

### KURZBESCHREIBUNG DER ZEICHNUNGEN

Weitere Merkmale und Vorteile der Erfindung ergeben sich aus den Ansprüchen und aus der nachfolgenden Beschreibung von bevorzugten Ausführungsbeispielen der Erfindung, die nachfolgend anhand der Figuren erläutert sind. Dabei zeigen:
- Fig. 1: schematisch eine Ausführungsform eines erfindungsgemäßen Knochenimplantats,
- Fig. 2: schematisch eine weitere Ausführungsform eines erfindungsgemäßen Knochenimplantats und
- Fig. 3: schematisch eine weitere Ausführungsform eines erfindungsgemäßen Knochenimplantats.

### DETAILLIERTE FIGURENBESCHREIBUNG

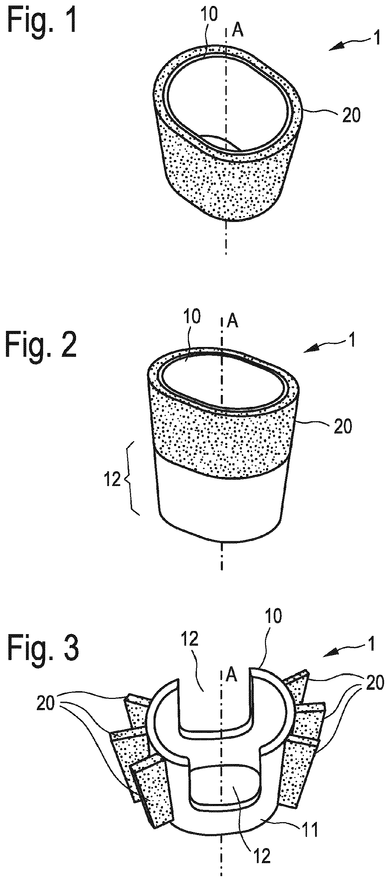

Das in Fig. 1 schematisch dargestellte Knochenimplantat 1 weist einen Grundkörper 10 sowie einen Ummantelungskörper 20 auf.

Der Grundkörper 10 ist in Form eines in axialer Richtung A beidseitig offenen oder geöffneten und vorzugsweise konusförmigen Hohlkörpers gestaltet.

Der Grundkörper 10 weist ein lasttragendes Material auf oder besteht aus einem lasttragenden Material. Bei dem lasttragenden Material handelt es sich vorzugsweise um ein in vivo nicht abbaubares/in vivo nicht resorbierbares Material, wie beispielsweise eine Titanlegierung oder einen Kunststoff, wie beispielsweise Polyetheretherketon. Dadurch ist mit besonderem Vorteil sowohl eine ausreichende Primär- als auch Sekundärstabilität des Knochenimplantats 1 nach dessen Platzierung an/in einem Knochendefekt erzielbar. Bezüglich weiterer geeigneter Materialien für den Grundkörper 10 wird auf die allgemeine Beschreibung Bezug genommen.

Bevorzugt wird der Grundkörper 10, wie in Fig. 1 dargestellt, vollständig von dem Ummantelungskörper 20 umgeben oder ummantelt. Der Ummantelungskörper 20 weist ein in vivo abbaubares/in vivo resorbierbares Material auf oder besteht aus einem solchen Material. Bei dem in vivo abbaubaren/in vivo resorbierbaren Material kann es sich beispielsweise um ein Calciumphosphat, insbesondere um β-Tricalciumphosphat und/oder Hydroxylapatit, handeln. Alternativ kann es sich bei dem in vivo abbaubaren/in vivo resorbierbaren Material um ein Polymer, wie beispielsweise Polycaprolacton, oder um ein Metall, wie beispielsweise Magnesium, handeln. Bezüglich weiterer geeigneter Materialien für den Ummantelungskörper 20 wird auf die allgemeine Beschreibung Bezug genommen.

Durch den (wenigsten teilweise) in vivo abbaubar/in vivo resorbierbar ausgebildeten Ummantelungskörper 20 ist mit besonderem Vorteil eine biologische Rekonstruktion eines zu behandelnden oder versorgenden Knochendefekts, wie beispielsweise eines tibialen oder femoralen Knochendefekts, erzielbar. Insbesondere von Vorteil ist, dass durch die biologische Rekonstruktion die Größe des Knochendefekts minimiert werden kann. Hierdurch sinkt das Risiko einer etwaigen Revisionsoperation. Gegebenenfalls werden durch die Verringerung der Knochendefektgröße günstigere Ausgangsbedingungen für eine erfolgreiche Durchführung von Revisionsoperationen erzeugt.

Der Grundkörper 10 kann beispielsweise eine Wanddicke von 3 mm bis 5 mm aufweisen, während der Ummantelungskörper 20 beispielsweise eine Manteldicke von 2 mm bis 10 mm aufweisen kann.

Der Ummantelungskörper 20 kann dabei, wie in Fig. 1 dargestellt, eine gleichmäßige Dicke aufweisen. Alternativ kann der Ummantelungskörper, insbesondere in axialer Richtung oder Längsrichtung, eine ungleichmäßige Dicke aufweisen (nicht dargestellt). Dadurch kann insbesondere asymmetrisch ausgebildeten Knochendefektstrukturen Rechnung getragen werden.

Ferner kann der Ummantelungskörper 20 eine offenporige Oberfläche aufweisen. Alternativ kann der Ummantelungskörper 20 vollständig oder durchgehend offenporig gestaltet sein. Durch die beiden vorgenannten Ausführungsformen für den Ummantelungskörper 20 ist mit besonderem Vorteil eine Osteointegration, d.h. ein An- und/oder Einwachsen von Knochengewebe an und/oder in den Ummantelungskörper 20 hinein, erzielbar. Dies wiederum trägt zu einer zusätzlichen Verbesserung der Primär- wie Sekundärstabilität des Knochenimplantats 1 bei.

Ferner weist der Ummantelungskörper, insbesondere bereichs- oder schichtweise, unterschiedliche in vivo abbaubare/in vivo resorbierbare Materialien auf, welche sich in Bezug auf ihre in vivo Abbaugeschwindigkeit/in vivo Resorptionsgeschwindigkeit voneinander unterscheiden. Dadurch sind mit besonderem Vorteil unterschiedliche Abbau-/Resorptionskinetiken des Ummantelungskörpers 20 und insbesondere eine Steuerung der mechanischen Stabilität des Knochenimplantats 1, der Knochenstimulierung sowie des Knochenwachstums möglich.

Fig. 2 zeigt eine weitere Ausführungsform eines erfindungsgemäßen Knochenimplantats 1.

Das Knochenimplantat 1 weist einen Grundkörper 10 sowie einen den Grundkörper 10 nur teilweise umgebenden Ummantelungskörper 20 auf.

Der Grundkörper 10 liegt in Form eines in axialer Richtung A beidseitig offenen oder geöffneten, vorzugsweise konusförmigen Hohlkörpers vor. Der Grundkörper 10 weist ein lasttragendes Material auf oder besteht aus einem lasttragenden Material. Bei dem lasttragenden Material handelt es sich vorzugsweise um ein in vivo nicht abbaubares/in vivo nicht resorbierbares Material, wie beispielsweise um eine Titanlegierung, insbesondere um die Titanlegierung Ti-6Al-4V, handeln. Hierbei handelt es sich um eine Titanlegierung, welche Titan, Aluminium und Vanadium sowie optional Verunreinigungen, insbesondere in Form von Eisen und/oder Sauerstoff und/oder Kohlenstoff und/oder Stickstoff und/oder Wasserstoff, aufweist oder aus Titan, Aluminium und Vanadium sowie optional Verunreinigungen, insbesondere in Form von Eisen und/oder Sauerstoff und/oder Kohlenstoff und/oder Stickstoff und/oder Wasserstoff, besteht. Bevorzugt weist die Titanlegierung neben Titan einen Aluminiumanteil von 5,5 Massenprozent bis 6,75 Massenprozent, insbesondere 6 Massenprozent, einen Vanadiumanteil von 3,5 Massenprozent bis 4,5 Massenprozent, insbesondere 4 Massenprozent, sowie optional einen Eisenanteil von 0,4 Massenprozent und/oder einen Sauerstoffanteil von 0,2 Massenprozent und/oder einen Kohlenstoffanteil von 0,08 Massenprozent und/oder einen Stickstoffanteil von 0,05 Massenprozent und/oder einen Wasserstoffanteil von 0,015 Massenprozent auf oder besteht aus den vorgenannten Elementen/Bestandteilen. Insbesondere kann der Grundkörper 10 eine Gitterstruktur, vorzugsweise aus Ti-6Al-4V, besitzen. Bezüglich weiterer geeigneter Materialien für den Grundkörper 10 wird auf die allgemeine Beschreibung sowie auf die Beschreibung zur Fig. 1 Bezug genommen.

Durch eine nur teilweise Ummantelung des Grundkörpers 10 mit dem Ummantelungskörper 20 ist vorteilhafterweise eine anwendungsorientierte Steuerung der Dicke des Knochenimplantats 1 möglich. Bevorzugt ist der nicht ummantelte Bereich 12 des Knochenimplantats 1 dazu vorgesehen, einen (nicht dargestellten) Verlängerungsschaft eines Gelenkimplantats aufzunehmen. Handelt es sich bei dem Knochenimplantat 1 um ein tibiales Knochenimplantat, stellt der nicht ummantelte Abschnitt 12 einen proximalen Abschnitt des Knochenimplantats dar. Handelt es sich bei dem Knochenimplantat 1 dagegen um ein femorales Knochenimplantat, stellt der nicht ummantelte Abschnitt 12 einen distalen Abschnitt des Knochenimplantats dar.

Bezüglich weiterer Merkmale und Vorteile des Knochenimplantats 1, insbesondere des Grundkörpers 10 sowie des Ummantelungskörpers 20, wird zur Vermeidung von Wiederholungen vollständig auf die Beschreibung zur Fig. 1 Bezug genommen. Die dort in Bezug auf das Knochenimplantat 1, insbesondere den Grundkörper 10 sowie den Ummantelungskörper 20, beschriebenen Merkmale und Vorteile gelten sinngemäß auch für das in Fig. 2 dargestellte Knochenimplantat 1.

Fig. 3 zeigt eine weitere Ausführungsform eines erfindungsgemäßen Knochenimplantats 1.

Das in Fig. 3 schematisch dargestellte Knochenimplantatsystem 1 weist einen Grundkörper 10 sowie eine Vielzahl von Formkörpern 20 auf. Dargestellt sind beispielhaft sechs Formkörper 20. Es versteht sich, dass das Knochenimplantat 1 aber auch weniger oder mehr Formkörper aufweisen kann.

Der Grundkörper 10 ist als ein in axialer Richtung A beidseitig offener oder geöffneter konusförmiger Hohlkörper gestaltet.

Der Grundkörper 10 weist ein lasttragendes Material, insbesondere eine Titanlegierung, wie beispielsweise Ti-6Al-4V, oder einen Kunststoff, insbesondere Polyetheretherketon, auf oder besteht aus einem solchen Material. Dadurch ist mit besonderem Vorteil eine ausreichende Primär- wie Sekundärstabilität erzielbar. Bezüglich weiterer geeigneter Materialien für den Grundkörper 10 wird auf die allgemeine Beschreibung sowie die Beschreibung zu den Fig. 1 und 2 Bezug genommen.

Vorzugsweise weist der Grundkörper 10 eine Wandung 11 mit zwei vorzugsweise gegenüberliegend ausgebildeten Durchbrüchen oder Aussparungen 12 auf. Die Durchbrüche bzw. Aussparungen 12 sind vorzugsweise u-förmig gestaltet.

Die Formkörper 20 weisen ein in vivo abbaubares/in vivo resorbierbares Material auf oder bestehen aus einem solchen Material. Dadurch lässt sich mit besonderem Vorteil eine biologische Rekonstruktion eines Knochendefekts erzielen. Durch die biologische Rekonstruktion des Knochendefekts kommt es vorteilhafterweise zu einer Minimierung der Knochendefektgröße. Dies wiederum reduziert das Risiko von Revisionsoperationen und/oder schafft günstige Voraussetzungen für eine erfolgreiche Durchführung von Revisionsoperationen. Bei dem in vivo abbaubaren/in vivo resorbierbaren Material kann es sich beispielsweise um ein Calciumphosphat, insbesondere um β-Tricalciumphosphat und/oder Hydroxylapatit, handeln. Alternativ kann es sich bei dem in vivo abbaubaren/in vivo resorbierbaren Material um ein Polymer, wie beispielsweise Polycaprolacton, oder um ein Metall, wie beispielsweise Magnesium, handeln. Bezüglich weiterer geeigneter Materialien für den Ummantelungskörper 20 wird auf die allgemeine Beschreibung sowie auf die Beschreibung zu den Fig. 1 und 2 Bezug genommen.

Die Formkörper 20 ragen in radialer Richtung vom Grundkörper 10 ab. Die Formkörper 20 sind stoffschlüssig mit dem Grundkörper 10 verbunden bzw. stoffschlüssig an diesem angefügt.

Beispielsweise können die Formkörper 20 mit dem Grundkörper 10 verklebt oder verschweißt sein. Alternativ können die Formkörper 20 mittels eines generativen Fertigungsverfahrens außenseitig, d.h. an einer Außenfläche 12 des Grundkörpers 10, angeformt sein.

Die Formkörper 20 sind vorzugsweise als Flächengebilde, insbesondere in Form von Platten oder Scheiben, gestaltet. Dies bedeutet, dass die Formkörper 20 eine Länge und eine Breite aufweisen, die kleiner sind als eine Dicke (Höhe) der Formkörper 20. Bevorzugt besitzen die Formkörper 20 jeweils zwei gegenüberliegend angeordnete Hauptflächen, welche jeweils von zwei gegenüberliegend angeordneten Querseitenflächen sowie von zwei gegenüberliegend angeordneten Längsseitenflächen begrenzt sind.

Zur Verbesserung der Osteointegration des Knochenimplantats 1 kann es ferner bevorzugt sein, wenn die Formkörper 20 jeweils eine poröse, insbesondere offenporige, und/oder mikrostrukturierte Oberfläche aufweisen oder aus einem porösen, insbesondere offenporigen, Material bestehen. Bezüglich eines insoweit geeigneten Materials wird ebenfalls auf die allgemeine Beschreibung sowie auf die Beschreibung zu den Fig. 1 und 2 Bezug genommen.

## Patentansprüche

1. Knochenimplantat (1), aufweisend
- einen Grundkörper (10) in Form eines in axialer Richtung (A) beidseitig offenen Hohlkörpers, aufweisend oder bestehend aus einem lasttragenden Material, und
- einen Ummantelungskörper (20), der den Grundkörper (10) außenseitig wenigstens teilweise ummantelt und ein in vivo abbaubares/in vivo resorbierbares Material aufweist oder aus einem in vivo abbaubaren/in vivo resorbierbaren Material besteht, oder eine Vielzahl von Formkörpern (20), die in radialer Richtung vom Grundkörper (10) abstehen und ein in vivo abbaubares/in vivo resorbierbares Material aufweisen oder aus einem in vivo abbaubaren/in vivo resorbierbaren Material bestehen, **dadurch gekennzeichnet, dass** der Ummantelungskörper (20) beziehungsweise die Formkörper (20) jeweils wenigstens zwei unterschiedliche in vivo abbaubare/in vivo resorbierbare Materialien aufweisen, die sich in Bezug auf ihre in vivo Abbaugeschwindigkeit/in vivo Resorptionsgeschwindigkeit voneinander unterscheiden.

2. Knochenimplantat (1) nach Anspruch 1, wobei der Grundkörper (10) einen eckenlosen, insbesondere kreisförmigen, ovalen oder elliptischen, Querschnitt aufweist.

3. Knochenimplantat (1) nach Anspruch 1 oder 2, wobeider Grundkörper (10) konusförmig gestaltet ist und einen sich wenigstens abschnittsweise verjüngenden Innendurchmesser aufweist.

4. Knochenimplantat (1) nach einem der vorhergehenden Ansprüche, wobei der Grundkörper (10) eine Wanddicke von 1 mm bis 30 mm, insbesondere 5 mm bis 20 mm, vorzugsweise 8 mm bis 15 mm, aufweist.

5. Knochenimplantat (1) nach einem der vorhergehenden Ansprüche, wobei es sich bei dem lasttragenden Material des Grundkörpers (10) um ein in vivo nicht abbaubares/in vivo nicht resorbierbares Material handelt.

6. Knochenimplantat (1) nach einem der vorhergehenden Ansprüche, insbesondere nach Anspruch 5, wobei das lasttragende Material, insbesondere das in vivo nicht abbaubare/in vivo nicht resorbierbare Material, ausgewählt ist aus der Gruppe bestehend aus Metalle wie Titan und/oder Tantal, Legierungen wie Titanlegierungen, Keramiken, Kunststoffe, Polyetherketone Polyetheretherketon, Polyetherketonketon, Polyetheretheretherketon, Polyetheretherketonketon, Polyetherketonetherketonketon, Polyolefine und Kombinationen davon.

7. Knochenimplantat (1) nach einem der vorhergehenden Ansprüche, wobei der Ummantelungskörper (20) als Schicht oder Beschichtung auf dem Grundkörper (10) ausgebildet ist.

8. Knochenimplantat (1) nach einem der vorhergehenden Ansprüche, wobei der Ummantelungskörper (20) eine Dicke von 0,1 mm bis 30 mm, insbesondere 1 mm bis 10 mm, vorzugsweise 2 mm bis 5 mm, aufweist.

9. Knochenimplantat (1) nach einem der vorhergehenden Ansprüche, wobei der Ummantelungskörper (20) eine in axialer Richtung (A) ungleichmäßige Form und/oder Dicke aufweist.

10. Knochenimplantat (1) nach Anspruch 9, wobei die Dicke des Ummantelungskörpers (20) an einem Ende des Ummantelungskörpers (20) größer ist als einem anderen Ende des Ummantelungskörpers (20) und/oder die Dicke des Ummantelungskörpers (20) in Richtung eines Endes des Ummantelungskörpers (20) zunimmt.

11. Knochenimplantat (1) nach einem der vorhergehenden Ansprüche, wobei der Grundkörper (10) außenseitig teilweise nicht von dem Ummantelungskörper (20) ummantelt ist.

12. Knochenimplantat (1) nach einem der vorhergehenden Ansprüche, wobei der Grundkörper (10) und/oder der Ummantelungskörper (20) und/oder die Formkörper (20) eine poröse, insbesondere offenporige, und/oder mikrostrukturierte Oberfläche aufweisen.

13. Knochenimplantat (1) nach einem der vorhergehenden Ansprüche, wobei die wenigstens zwei unterschiedlichen in vivo abbaubaren/in vivo resorbierbaren Materialien ausgewählt sind aus der Gruppe bestehend aus Polyhydroxyalkanoate wie Polylactid und/oder Polyglycolid, Polycaprolacton, Calciumphosphate wie β-Tricalciumphosphat und/oder Hydroxylapatit und Kombinationen davon.

14. Verfahren zum Herstellen eines Knochenimplantats (1) nach einem der vorhergehenden Ansprüche, **dadurch gekennzeichnet, dass** ein Grundkörper (10) in Form eines in axialer Richtung (A) beidseitig offenen Hohlkörpers, aufweisend oder bestehend aus einem lasttragenden Material, mittels eines generativen Fertigungsverfahrens wenigstens teilweise mit einem Ummantelungskörper (20), aufweisend oder bestehend aus einem in vivo abbaubaren/in vivo resorbierbaren Material, ummantelt wird oder mit einer Vielzahl von Formkörpern (20), aufweisend oder bestehend aus einem in vivo abbaubaren/in vivo resorbierbaren Material, derart verbunden wird, dass die Formkörper (20) in radialer Richtung (A) von dem Grundkörper (10) abstehen.

## Claims

1. A bone implant comprising
- a main body in the form of a hollow body open on both sides in the axial direction, comprising or consisting of a load-bearing material,
and
- an encasing body which at least partially encases the main body on the outside and comprises an in vivo degradable/in vivo resorbable material or consists of an in vivo degradable/in vivo resorbable material, or a multiplicity of shaped bodies which protrude from the main body in the radial direction and comprise an in vivo degradable/in vivo resorbable material or consist of an in vivo degradable/in vivo resorbable material, **characterized in that** the encasing body and the shaped bodies, respectively each comprise at least two different in vivo degradable/in vivo resorbable materials which differ from one another with regard to their in vivo degradation rate/in vivo resorption rate.

2. The bone implant as claimed in claim 1, **characterized in that** the main body has a corner-free, in particular circular, oval or elliptical, cross section.

3. The bone implant as claimed in claim 1 or 2, **characterized in that** the main body is conical and has an at least sectionally tapering inner diameter.

4. The bone implant as claimed in any of the preceding claims, **characterized in that** the main body has a wall thickness of 1 mm to 30 mm, in particular 5 mm to 20 mm, preferably 8 mm to 15 mm.

5. The bone implant as claimed in any of the preceding claims, **characterized in that** the load-bearing material of the main body is an in vivo nondegradable/in vivo nonresorbable material.

6. The bone implant as claimed in any of the preceding claims, in particular as claimed in claim 5, **characterized in that** the load-bearing material, in particular the in vivo nondegradable/in vivo nonresorbable material, is selected from the group consisting of metals such as titanium and/or tantalum, alloys such as titanium alloys, ceramics, plastics, polyetherketones, polyetheretherketone, polyetherketoneketone, polyetheretheretherketone, polyetheretherketoneketone, polyetherketoneetherketoneketone, polyolefins and combinations thereof.

7. The bone implant as claimed in any of the preceding claims, **characterized in that** the encasing body is in the form of a layer or coating on the main body.

8. The bone implant as claimed in any of the preceding claims, **characterized in that** the encasing body has a thickness of 0.1 mm to 30 mm, in particular 1 mm to 10 mm, preferably 2 mm to 5 mm.

9. The bone implant as claimed in any of the preceding claims, **characterized in that** the encasing body has a nonuniform shape and/or thickness in the axial direction.

10. The bone implant as claimed in claim 9, **characterized in that** the thickness of the encasing body is greater at one end of the encasing body than another end of the encasing body and/or the thickness of the encasing body increases toward one end of the encasing body.

11. The bone implant as claimed in any of the preceding claims, **characterized in that** the main body is partially not encased by the encasing body on the outside.

12. The bone implant as claimed in any of the preceding claims, **characterized in that** the main body and/or the encasing body and/or the shaped bodies have a porous, in particular open-pored, and/or microstructured surface.

13. The bone implant as claimed in any of the preceding claims, **characterized in that** the at least two different in vivo degradable/in vivo resorbable materials are selected from the group consisting of polyhydroxyalkanoates such as polylactide and/or polyglycolide, polycaprolactone, calcium phosphates such as β-tricalcium phosphate and/or hydroxyapatite and combinations thereof.

14. A method for producing a bone implant as claimed in any of the preceding claims, **characterized in that** a main body in the form of a hollow body open on both sides in the axial direction, comprising or consisting of a load-bearing material, is, by means of an additive manufacturing process, at least partially encased by an encasing body comprising or consisting of an in vivo degradable/in vivo resorbable material or connected to a multiplicity of shaped bodies comprising or consisting of an in vivo degradable/in vivo resorbable material in such a way that the shaped bodies protrude from the main body in the radial direction.

## Revendications

1. Implant osseux (1), présentant
- un corps de base (10) sous la forme d'un corps creux ouvert des deux côtés dans la direction axiale (A), présentant ou constitué d'un matériau porteur de charge, et
- un corps d'enveloppe (20) qui enveloppe au moins partiellement le corps de base (10) du côté extérieur et présente un matériau dégradable in vivo/résorbable in vivo ou est constitué d'un matériau dégradable in vivo/résorbable in vivo, ou une pluralité de corps façonnés (20) qui font saillie à partir du corps de base (10) dans la direction radiale et présentent un matériau dégradable in vivo/résorbable in vivo ou sont constitués d'un matériau dégradable in vivo/résorbable in vivo, **caractérisé en ce que** le corps d'enveloppe (20) ou les corps façonnés (20) présentent respectivement au moins deux matériaux différents dégradables in vivo/résorbables in vivo qui diffèrent les uns des autres en ce qui concerne leur vitesse de dégradation in vivo/vitesse de résorption in vivo.

2. Implant osseux (1) selon la revendication 1, dans lequel le corps de base (10) présente une section transversale sans angles, en particulier circulaire, ovale ou elliptique.

3. Implant osseux (1) selon la revendication 1 ou 2, dans lequel le corps de base (10) est formé de manière conique et présente un diamètre intérieur se rétrécissant au moins dans certaines régions.

4. Implant osseux (1) selon l'une des revendications précédentes, dans lequel le corps de base (10) présente une épaisseur de paroi de 1 mm à 30 mm, en particulier de 5 mm à 20 mm, de préférence de 8 mm à 15 mm.

5. Implant osseux (1) selon l'une des revendications précédentes, dans lequel le matériau porteur de charge du corps de base (10) est un matériau non dégradable in vivo/non résorbable in vivo.

6. Implant osseux (1) selon l'une des revendications précédentes, en particulier selon la revendication 5, dans lequel le matériau porteur de charge, en particulier le matériau non dégradable in vivo/non résorbable in vivo, est sélectionné parmi le groupe constitué de métaux tels que le titane et/ou le tantale, d'alliages tels que des alliages de titane, de céramiques, de matériaux synthétiques, de polyéthercétones, de polyétheréthercétone, de polyéthercétonecétone, de polyétherétheréthercétone, de polyétheréthercétonecétone, de polyéthercétoneéthercétonecétone, de polyoléfines et de combinaisons de ceux-ci.

7. Implant osseux (1) selon l'une des revendications précédentes, dans lequel le corps d'enveloppe (20) est réalisé sous forme de couche ou de revêtement sur le corps de base (10).

8. Implant osseux (1) selon l'une des revendications précédentes, dans lequel le corps d'enveloppe (20) présente une épaisseur 0,1 mm à 30 mm, en particulier de 1 mm à 10 mm, de préférence de 2 mm à 5 mm.

9. Implant osseux (1) selon l'une des revendications précédentes, dans lequel le corps d'enveloppe (20) présente une forme et/ou une épaisseur non uniforme(s) dans la direction axiale (A).

10. Implant osseux (1) selon la revendication 9, dans lequel l'épaisseur du corps d'enveloppe (20) est plus grande à une extrémité du corps d'enveloppe (20) qu'à une autre extrémité du corps d'enveloppe (20) et/ou l'épaisseur du corps d'enveloppe (20) augmente en direction d'une extrémité du corps d'enveloppe (20).

11. Implant osseux (1) selon l'une des revendications précédentes, dans lequel le corps de base (10) est partiellement non enveloppé par le corps d'enveloppe (20) du côté extérieur.

12. Implant osseux (1) selon l'une des revendications précédentes, dans lequel le corps de base (10) et/ou le corps d'enveloppe (20) et/ou les corps façonnés (20) présentent une surface poreuse, en particulier à pores ouverts, et/ou microstructurée.

13. Implant osseux (1) selon l'une des revendications précédentes, dans lequel les au moins deux matériaux différents dégradables in vivo/résorbables in vivo sont sélectionnés parmi le groupe constitué de polyhydroxyalcanoates tels que du polylactide et/ou du polyglycolide, de polycaprolactone, de phosphates de calcium tels que du phosphate β-tricalcique et/ou d'hydroxylapatite et de combinaisons de ceux-ci.

14. Procédé permettant de produire un implant osseux (1) selon l'une des revendications précédentes, **caractérisé en ce qu'**un corps de base (10) sous la forme d'un corps creux ouvert des deux côtés dans la direction axiale (A), présentant ou constitué d'un matériau porteur de charge, est enveloppé à l'aide d'un procédé de fabrication générative au moins partiellement par un corps d'enveloppe (20), présentant ou constitué d'un matériau dégradable in vivo/résorbable in vivo ou relié à une pluralité de corps façonnés (20), présentant ou constitués d'un matériau dégradable in vivo/résorbable in vivo, de telle sorte que les corps façonnés (20) font saillie à partir du corps de base (10) dans la direction radiale (A).
